# EUROPÄISCHE PATENTANMELDUNG

(11) **EP 3 498 759 A1**
(43) Veröffentlichungstag der Anmeldung: **19.06.2019**
(21) Anmeldenummer: 17206928.8
(22) Anmeldetag: 13.12.2017
(51) Int. Cl.: C08G 69/16, C07D 225/02, C07D 201/04

(54) **VERFAHREN ZUR HERSTELLUNG VON POLYMEREN AUS MONOMEREN UMFASSEND LAURINLACTAM**

(71) Anmelder: Evonik Degussa GmbH, 45128 Essen (DE)
(72) Erfinder: Die Erfindernennung liegt noch nicht vor

(57) **Zusammenfassung**

Die vorliegende Erfindung betrifft Verfahren zur Herstellung von Polymeren aus Monomeren umfassend Laurinlactam, umfassend die Schritte
a. Beckmann-Umlagerung von Cyclododecanoxim zu Laurinlactam in Gegen-wart eines Beckmann-Umlagerungskatalysators,
b. Entfernung von Verunreinigungen aus dem Laurinlactam unter Erhalt aufge-reinigten Laurinlactams, und
c. Polymerisation von Monomeren umfassend aufgereinigtes Laurinlactam.

Zur Vermeidung von Verfärbungen oder Vergilbungen unter Alterungsbedingungen werden vor der Polymerisation polycyclische Stoffe enthaltend 24 Kohlenstoffatome und zumindest ein Heteroatom ausgewählt aus Sauerstoff und Stickstoff, die eine Molmasse zwischen 300 und 380 g/mol aufweisen, auf 500 ppm, bezogen auf Laurinlactam, begrenzt.

## Beschreibung

Die vorliegende Erfindung betrifft ein Verfahren zur Herstellung von Polymeren aus Monomeren umfassend Laurinlactam und ggf. weiteren Co-Monomeren sowie Polymere, die aus dem Verfahren erhalten werden.

Bekannte Polymere aus Monomeren umfassend Laurinlactam sind beispielsweise Polyamid 12, Copolyamide enthaltend Laurinlactam wie Caprolactam-Laurinlactam-Copolyamid oder BlockCopolymere wie Polyether-Block-Amide (PEBA). Laurinlactam wird beispielsweise aus Cyclododecanonoxim erhalten. Cyclododecanonoxim kann durch die Reaktion von Cyclododecanon und Hydroxylamin in einem organischen Lösungsmittel hergestellt werden (EP 2551261). Weiterhin kann Cyclododecanonoxim durch die Reaktion von Cyclododecanon mit Wasserstoffperoxid und Ammoniak in Anwesenheit eines Titansilikalitkatalysators gewonnen werden (EP 1316545 = US 2003/100795, EP 1663955 = US 2008/249300). Cyclododecanonoxim kann alternativ durch Fotooximierung von Cyclododecan erhalten werden (EP 0993438 = US 6197999).

Die Herstellung von Laurinlactam kann mittels Beckmann-Umlagerung von Cyclododecanonoxim zu Laurinlactam in Anwesenheit einer starken Säure, Cyanurchlorid, Phosphortrichlorid oder Thionylchlorid erfolgen (Ullmann's encyclopedia 2009, 11, 33-36; EP 2013162 = US 2009/306367; EP2123635). Als starke Säure kann beispielweise Schwefelsäure benutzt werden.

Für viele Anwendungen ist die Farbe des Polymers sehr wichtig. Aus dem Stand der Technik ist bekannt, dass Verunreinigungen im Laurinlactam die Vergilbung von entsprechenden Polyamiden begünstigen. In EP 1773765 (US 2008/071081) werden Lactame mittels Fotooximation hergestellt, wobei chlorierte Verunreinigungen erhalten werden. Bei der alternativen Herstellung von Laurinlactam ausgehend von Cyclododecanon (Beckmann-Umlagerung) entstehen keine chlorierten Verunreinigungen. Dennoch werden Vergilbungen des Polymers aus diesem Laurinlactam beobachtet.

Die Farbe bzw. Vergilbung wird üblicherweise mittels Spektralphotometer ermittelt, wobei eine Quantifizierung anhand des b-Werts vorgenommen wird. Außerdem wird der Farbabstand ΔE zwischen dem Polymer vor und nach Alterung gemessen. Je niedriger diese Werte sind, desto weniger Vergilbung weist das Polymer auf.

Die Aufgabe der vorliegenden Erfindung bestand darin, ein Verfahren zur Herstellung von Polymeren aus Monomeren umfassend Laurinlactam bereitzustellen, welches gegenüber dem Stand der Technik geringere Vergilbung zeigt. Das Laurinlactam sollte dabei über die heute üblicherweise in großtechnischem Maßstab eingesetzte Beckmann-Umlagerung des Cyclododecanoxims erhalten werden.

Es wurde nunmehr gefunden, dass die Anwesenheit von polycyclischen Stoffen mit 24 Kohlenstoffatomen und zumindest ein Heteroatom (Sauerstoff, Stickstoff) sowie mit einer Molmasse zwischen 300 und 380 g/mol in Laurinlactam die Farbe von laurinlactamhaltigen Polymeren wie Polyamid 12 beeinflusst. Solche Verunreinigungen entstehen während der Herstellung von Laurinlactam und verursachen maßgeblich die Vergilbung der Polymere nach Alterung (24 h bei 100 °C im Umlufttrockenschrank, Luftatmosphäre).

Demgemäß wurde ein Verfahren zur Herstellung von Polymeren aus Monomeren umfassend Laurinlactam gefunden, welches die folgenden Schritte umfasst:
a. Beckmann-Umlagerung von Cyclododecanoxim zu Laurinlactam in Gegenwart eines Beckmann-Umlagerungskatalysators,
b. Entfernung von Verunreinigungen aus dem Laurinlactam unter Erhalt aufgereinigten Laurinlactams, und
c. Polymerisation von Monomeren umfassend aufgereinigtes Laurinlactam.

Die Verunreinigungen sind polycyclische Stoffe, welche 24 Kohlenstoffatome und zumindest ein Heteroatom ausgewählt aus Sauerstoff und Stickstoff umfassen sowie eine Molmasse zwischen 300 und 380 g/mol aufweisen, wobei die Verunreinigungen in Schritt b. auf 500 ppm, vorzugsweise 100 ppm, jeweils bezogen auf Laurinlactam, begrenzt werden. Die Obergrenze bezieht sich auf die Summe der zuvor definierten Verunreinigungen. Die Verunreinigungen weisen vorzugsweise zwischen 250 nm und 290 nm ein lokales Absorptionsmaximum im UV-Spektrum auf.

Es wurde überraschend gefunden, dass eine erhöhte Konzentration dieser Verunreinigungen im Laurinlactam die Farbe bzw. Vergilbung eines laurinlactamhaltigen Polymers nach Alterung deutlich beeinflusst. Laurinlactamhaltige Polymere, die nach dem erfindungsgemäßen Verfahren hergestellt wurden, wiesen nach Alterung einen b-Wert < 5 und einen ΔE-Wert < 5 auf. Die Werte wurden mit einem Color i7 Spektralfotometer der Firma X-Rite nach DIN 6174 gemessen; weitere Angaben finden sich in den Beispielen.

Im Sinne dieser Erfindung sind Polymere aus Monomeren umfassend Laurinlactam zu verstehen als Polymere, die zumindest aus Laurinlactam als Monomer erhalten werden. Definitionsgemäß werden von Laurinlactam verschiedene Monomere als Co-Monomere bezeichnet. Damit umfasst der Begriff "Polymer aus Monomeren umfassend Laurinlactam" Homopolyamide und Co-Polyamide. Polymere aus Monomeren umfassend Laurinlactam sind insbesondere das Homopolyamid 12 oder Copolyamide wie PEBA oder Caprolactam-Laurinlactam-Copolyamide. Bevorzugte Polymere enthalten mindestens 20 Gew.-% Laurinlactam, besonders bevorzugt 30 Gew.-%, jeweils bezogen auf das Gesamtgewicht des Polymers. Ein ganz besonders bevorzugtes Polymer ist Polyamid 12.

Ein geeigneter Beckmann-Umlagerungskatalysator wird ausgewählt aus einer Säure mit pKs ≤ 0, Cyanurchlorid, Phosphortrichlorid, Thionylchlorid oder Mischungen daraus, wobei Schwefelsäure bevorzugt ist.

Die Polymerisation des aufgereinigten Laurinlactams sowie ggf. weiterer Monomere (Co-Monomere) erfolgt vorzugsweise bei einer Temperatur zwischen 250 °C und 350 °C. Die optionalen Co-Monomere werden für laurinlactamhaltige Polymere wie Copolyamide oder Polyether-blockamide benötigt. Der bevorzugte Temperaturbereich liegt zwischen 270 °C und 330 °C, besonders bevorzugt sind 280 °C bis 300 °C. Die Polymerisation wird vorzugsweise in Anwesenheit von Wasser vorgenommen, wobei der Wassergehalt bevorzugt 2 bis 51 Gew.-%, bezogen auf das Gesamtgewicht aus Wasser und Monomeren, beträgt. Besonders bevorzugt werden 3 bis 15 Gew.-% Wasser eingesetzt. In einer bevorzugten Ausführungsform wird die Polymerisation bei einer Temperatur zwischen 250 °C und 350 °C in Gegenwart von 2 bis 51 Gew.-% Wasser durchgeführt.

Geeignete Co-Monomere werden ausgewählt aus Lactamen mit 6 bis 14 C-Atomen, insbesondere Caprolactam, Aminocarbonsäuren mit 6 bis 14 C-Atomen, Diaminen mit 4 bis 23 C-Atomen, Dicarbonsäuren mit 4 bis 23 C-Atomen und Polyether mit zwei terminalen Gruppen. Polyether können beispielweise Polyethylenglykole, Polypropylenglykole oder Polytetramethylenetherglykole sein. Terminale Endgruppen der Polyether sind beispielsweise Hydroxyl-, Amino- und Carboxylgruppen. Vorzugsweise haben die Polyether zwei terminale Hydroxylgruppen, zwei terminale Aminogruppen, zwei terminale Carboxylgruppen oder je eine terminale Hydroxyl- und Aminogruppe. Ein aus dem Verfahren erhaltenes Homopolyamid oder Copolyamid kann anschließend erneut mit Polyethern mit zwei terminalen Gruppen polymerisiert werden.

Die Polymerisation kann im Batchverfahren oder im kontinuierlichen Verfahren durchgeführt werden, wobei ein kontinuierliches Verfahren bevorzugt ist. Sie kann ohne Katalysator oder unter Zugabe einer katalytischen Menge einer anorganischen Säure durchgeführt werden. Als anorganische Säure eignen sich beispielweise H₃PO₄ oder H₃PO₂.

Die Verunreinigungen, die nach der Beckmann-Umlagerung vorliegen, können mittels Gaschromatographie-Massenspektrometrie (GC-MS) bestimmt werden. Die Massenspektrometrie ermöglicht die Ermittlung der molaren Masse und der Summenformel. Mittels GC wird der Anteil an Verunreinigungen im Laurinlactam ermittelt. Die Bestimmungen wurden mit einem Triplus Autosampler mit Trace GC 1310 und QExactive Orbitrap durchgeführt. Die Trennung wurde mit einer Säule TG 5SilMS von 30 m Länge und 0,25 mm Durchmesser vorgenommen. Das Temperaturprogramm entsprach 1 min bei 60 °C, dann Aufheizen bis 220 °C (8K/min), dann Aufheizen bis 250 °C (1K/min) und 10 min bei 250 °C. Die MS Detektion wurde mittels Elektronenstoßionisation im Massenbereich 30 bis 500 g/mol und eine Massenauflösung R=60000 durchgeführt.

Weiterhin konnte mittels UV-Spektrometrie und ¹H-NMR und ¹³C-NMR nachgewiesen werden, dass zumindest ein großer Teil dieser Verunreinigungen im Laurinlactam ein lokales Absorptionsmaximum zwischen 250 und 290 nm aufweist. Zudem können die Verunreinigungen mindestens eine Doppelbindung und/oder einen aromatischen Ring beinhalten. UV-Spektren wurden mit einem HPLC Gerät System Accela 1050 von ThermoFisher Scientific gemessen. Dafür wurde eine Trennsäule 200 x 2mm Hypersil Gold C18, Partikelgröße 1.9 µm benutzt und eine Mischung aus Wasser, Trifluoressigsäure, Acetonitiril und n-Propanol als Laufmittel eingesetzt. Mit dem Photodiodenzeile- (PDA) Detektor des Geräts wurden UV-Spektren über die jeweiligen Peaks akkumuliert und Untergrund korrigiert. NMR Spektren wurden mit einem 500 MHz Spektrometer der Firma Bruker in CDCl₃ gemessen.

Aus den nachgewiesenen Summenformeln und den Hinweisen aus den UV- und NMR-Spektren wird vermutet, dass beispielweise die folgenden Strukturen Verunreinigungen im Sinne der Erfindung darstellen:

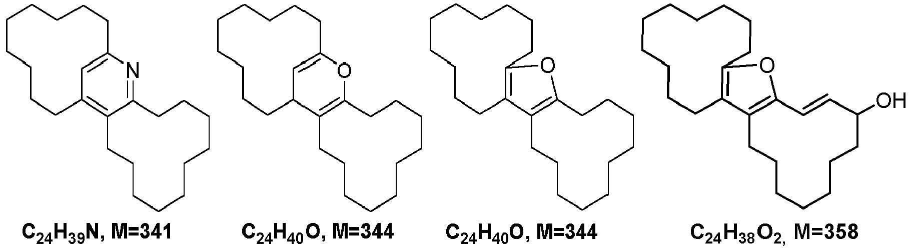

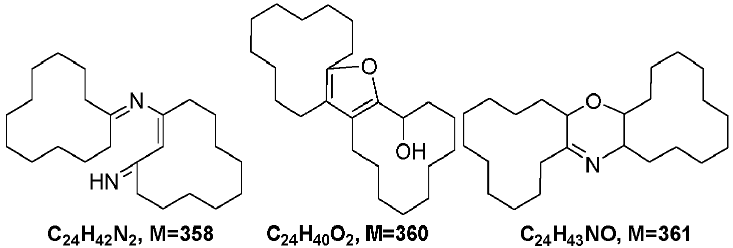

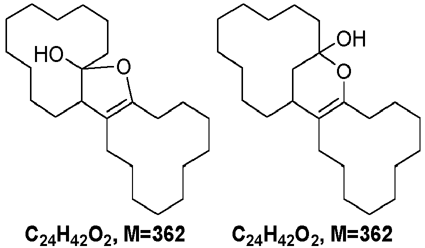

Die Verunreinigungen entstehen durch chemische Reaktionen zwischen zwei Verbindungen enthaltend zwölf C-Atome. Diese Verbindungen können beispielweise Cyclododecanon, Cyclododecanonoxim, Cyclododecenon (ungesättigtes Keton) oder Cyclododecenonoxim (ungesättigtes Oxim) umfassen. Die unerwünschten Nebenreaktionen können zum Beispiel während der Oximbildung, während der Beckmann-Umlagerung oder während der Destillation von Laurinlactam stattfinden.

Die Entfernung der Verunreinigungen aus dem Laurinlactam (Schritt b.) wird vorzugsweise mittels Kristallisation, Destillation oder Rektifikation vorgenommen. Hierbei ist es bei der Destillation bevorzugt, wenn diese mit mindestens fünf theoretischen Böden vorgenommen wird.

In einer besonders bevorzugten Ausführungsform der Erfindung lässt sich der Gehalt an Verunreinigungen im Laurinlactam zusätzlich durch eine Aufarbeitung von Cyclododecanoxim vor der Beckmann-Umlagerung verringern. Das Oxim wird hierzu einer Wäsche mit Wasser, einer Wäsche mit Lauge wie Natronlauge, einer Wäsche mit Säure wie Salz- oder Schwefelsäure, einer Hydrierung, einer Kristallisation oder einer Kombinationen davon unterzogen. Diese Behandlung des Oxims kann die Bildung von Verunreinigungen im Laurinlactam reduzieren, so dass die Aufreinigung des Laurinlactams anschließend effizienter wird.

Das Cyclododecanonoxim wird vorzugsweise aus Cyclododecanon erhalten. Verfahren zur Herstellung des Oxims sind die im Stand der Technik bekannten Verfahren aus Cyclododecanon und Hydroxylamin oder Wasserstoffperoxid mit Ammoniak.

Darüber hinaus bildet ein Polymer enthaltend Laurinlactam, hergestellt nach dem zuvor beschriebenen Verfahren, einen weiteren Gegenstand der Erfindung.

### Beispiel

In den nachfolgenden Beispielen wird die Herstellung von Polyamid 12 nach unterschiedlichen erfindungsmäßen Verfahren sowie nicht erfindungsgemäße Referenzbeispiele beschrieben.

Der im Folgenden verwendete Begriff "Verunreinigungen" bezieht sich auf die folgende Definition: Polycyclische Stoffe enthaltend 24 Kohlenstoffatome und zumindest ein Heteroatom ausgewählt aus Sauerstoff und Stickstoff, die Molmasse liegt zwischen 300 und 380 g/mol

### Beispiele A: Herstellung von Laurinlactam

### Beispiel A1: Herstellung von Laurinlactam mit H₂SO₄ und anschließende Destillation

3,44 kg einer 25 Gew.-% Lösung von Cyclododecanon in Hydrocumol wurden in einen 20 L-Reaktor bei 90 °C eingefüllt. Dann wurden 2,83 kg Ammoniaklösung (25 Gew.-% in Wasser), 171 g Ammoniumsulfat, 22 g Hostapur® (Emulgator), 15,2g Titansilikalit TS-1 und 1,84 kg Wasser zugegeben. Das zweiphasige Gemisch wurde intensiv gerührt und 550 g H₂O₂-Lösung (70 Gew.-% in Wasser) wurden über 6 h in den Reaktor zudosiert. Am Ende zeigte eine GC-Analyse die vollständige Umsetzung von Cyclododecanon in Cyclododecanoxim. Das Rühren wurde gestoppt, damit sich die Phasen trennen konnten. Die wässrige Phase wurde abgelassen und die organische Phase wurde zweimal bei 95 °C mit 5 kg Wasser gewaschen.

Zu der Lösung von Cyclododecanoxim in Hydrocumol aus dem letzten Schritt wurden dann 900 g konzentrierte Schwefelsäure zugegeben. Das zweiphasige Gemisch wurde 30 min bei 50 °C gerührt. Die Phasen wurden danach getrennt. Die untere Phase enthaltend Cyclododecanoxim und Schwefelsäure wurde dann im Reaktor bei 122 °C aufgeheizt, wobei ein Druck von 1,5 bar erreicht wurde. Das Reaktionsgemisch wurde während 2h gerührt, wobei das Cyclododecanonoxim vollständig zum Laurinlactam umgesetzt wurde. Anschließend wurden 900 g Cyclododecanon, 1,8 kg Hydrocumol und 2,7 kg Wasser zugegeben. Nach 15 min wurden die Phasen getrennt. Die organische Phase wurde dann in einer ersten Destillationskolonne mit 20 theoretischen Trennstufen bei 200 °C Sumpftemperatur und einem Druck von 120 mbar destilliert, bis der Sumpf keinen Hydrocumol mehr enthielt. Der Sumpf aus der ersten Kolonne wurde anschließend in einer zweiten Destillationskolonne mit 15 theoretischen Trennstufen bei 215 °C Sumpftemperatur und einem Druck von 15 mbar destilliert, bis der Sumpf kein Cyclododecanon mehr enthielt. Im Sumpf dieser Kolonne wurde 850 g rohes Laurinlactam erhalten. Dieses rohe Laurinlactam hatte eine Reinheit von 99,85 % und enthielt 900 ppm Verunreinigungen.

Das rohe Laurinlactam wurde in einer dritten Destillationskolonne mit 5 theoretischen Trennstufen bei 200 °C Sumpftemperatur und einem Druck von 10 mbar destilliert. Im Destillat wurde 810 g Laurinlactam erhalten. Das Laurinlactam hatte eine Reinheit von 99,95 % und enthielt lediglich 30 ppm an Verunreinigungen.

### Beispiel A2: Herstellung von Laurinlactam mit H₂SO₄ und anschließende Kristallisation

Das rohe Laurinlactam aus Beispiel A1 wurde in 2,85 kg Hydrocumol bei 100 °C aufgelöst. Die resultierende 23 Gew.-%ige Lösung von Laurinlactam in Hydrocumol wurde langsam über 10 h bis 20 °C abgekühlt, was zu einer Kristallisation des reinen Laurinlactams führte. Das Laurinlactam wurde dann abfiltriert und bei 100 °C unter Vakuum (1 mbar) getrocknet, wodurch 780 g reines Laurinlactam erhalten wurde. Das Laurinlactam hatte eine Reinheit von 99,99 % und enthielt unter 5 ppm (unter Nachweisgrenze des GC-Geräts) Verunreinigungen.

### Beispiel A3: Herstellung von Laurinlactam mit Cyanurchlorid und anschließender Destillation

Cyclododecanoxim wurde analog zu Beispiel A1 hergestellt.

Die Lösung von Cyclododecanoxim in Hydrocumol wurde im 20L-Glasreaktor durch Einleitung von N2 bei 100 °C während 12 h getrocknet. Anschließend wies die Lösung einen Wassergehalt unter 100 ppm auf (Karl-Fischer Titration). Das Gemisch wurde auf 120 °C aufgeheizt und 70 g einer 10 Gew.-%iger Lösung von Cyanurchlorid in Toluol wurde zugegeben. Der Gehalt an Cyanurchlorid entsprach 2 mol-% bezogen auf Cyclododecanonoxim. Die Mischung wurde während 2h gerührt, wobei das Cyclododecanonoxim vollständig zum Laurinlactam umgesetzt wurde. Anschließend wurde bei 95 °C eine 2,5 Gew.-%ige NaOH-Lösung in Wasser unter starker Rührung des zweiphasigen Gemisches solange zudosiert, bis der pH-Wert in der wässrigen Phase pH 11 entsprach. Dann wurden die Phasen getrennt und die wässrige Phase wurde abgelassen. Die organische Phase wurde dann mit einer 0,5 Gew.-%igen H₂SO₄-Lösung in Wasser gewaschen, bis der pH-Wert der wässrigen Phase zwischen 6 und 7 lag. Die Phasen wurden getrennt und die wässrige Phase wurde abgelassen.

Anschließend wurde 900 g Cyclododecanon zugegeben und die Mischung wurde in einer ersten Destillationskolonne mit 25 theoretischen Trennstufen bei 210 °C Sumpftemperatur und einem Druck von 100 mbar destilliert, bis der Sumpf keinen Hydrocumol und kein Toluol mehr enthielt. Der Sumpf aus der ersten Kolonne wurde anschließend in einer zweiten Destillationskolonne mit 25 theoretischen Trennstufen bei 190 °C Sumpftemperatur und einem Druck von 10 mbar destilliert, bis der Sumpf kein Cyclododecanon mehr enthielt. Im Sumpf dieser Kolonne wurde 845 g rohes Laurinlactam erhalten. Dieses rohe Laurinlactam hatte eine Reinheit von 99,46 % und enthielt 1800 ppm an Verunreinigungen.

Das rohe Laurinlactam wurde in einer dritten Destillationskolonne mit 15 Trennstufen bei 185 °C Sumpftemperatur und einem Druck von 5 mbar destilliert. Im Destillat wurde 780 g Laurinlactam erhalten. Das Laurinlactam hatte eine Reinheit von 99,95 % und enthielt lediglich 20 ppm Verunreinigungen.

### Beispiele B: Herstellung von Polyamid 12

### Beispiel B1 (erfindungsgemäß): Herstellung von farblosem Polyamid 12

10 g reines Laurinlactam aus dem Beispiel A1 (30 ppm Verunreinigungen) wurde zusammen mit 10 g Wasser, 0,12 g Dodecandisäure und 0,6 mg H₃PO₂ in einen 25 mL druckfesten Reaktor eingefüllt. Das Gemisch wird 15 min mit Stickstoff inertisiert und mit einem Metallbad auf 130 °C aufgeheizt. Dann wurde die Temperatur über 30 min bis 280 °C aufgeheizt. Dabei entstand ein Druck von 10 bar. Die Temperatur wurde während 2,5 h bei 280 °C gehalten, wobei ein Druck von 22 bar erreicht wurde. Dann wurde der Druck langsam über 30 min bis zum atmosphärischen Druck entspannt. Das Reaktionsgemisch wurde noch während 2,5 h mit N₂ bei 280 °C überleitet. Der Reaktor wurde anschließend bis Raumtemperatur abgekühlt und 10,1 g Polyamid 12 wurde erhalten.

Nach der Herstellung des Polyamids wurden auf einer Drehbank mit dem Abstechstahl planparallele Scheiben (Maßabweichung < 1/10) mit einem Durchmesser von 14 mm und einer Dicke von 2 mm aus dem erstarrten Schmelzekörper abgedreht. Einzelne Scheiben mit Lunkern wurden ausgesondert. Die Farbe der Scheiben wurde anschließend mit einem Color i7 Spektralfotometer der Firma X-Rite nach DIN 6174 gemessen. Die Messungen wurden mit Glanzeinschluss und 10 mm Blende und mit der Lichtart D65/10° durchgeführt. Die Farbmessung der Polyamid-Scheibe ergab einen b-Wert von 0,3.

Die Alterung der Proben wurde in einem Umlufttrockenschrank unter normalen atmosphärischen Bedingungen bei einer Temperatur von 100 °C für 24 h durchgeführt. Anschließend wurde die Farbe erneut gemessen und das Material wies einen b-Wert von 3,5 und einen ΔE-Wert von 4,2 auf.

### Beispiel B2 (erfindungsgemäß): Herstellung von farblosem Polyamid 12

10 g reines Laurinlactam aus dem Beispiel A2 (unter 5 ppm Verunreinigungen) wurde zusammen mit 10 g Wasser, 0,12 g Dodecandisäure und 0,6 mg H₃PO₂ in einen 25 mL druckfesten Reaktor eingefüllt. Das Gemisch wurde 15 min mit Stickstoff inertisiert und mit einem Metallbad auf 130 °C aufgeheizt. Dann wurde die Temperatur über 30 min bis 280 °C aufgeheizt. Dabei entstand ein Druck von 10 bar. Die Temperatur wurde während 2,5 h bei 280 °C gehalten, wobei ein Druck von 22 bar erreicht wurde. Dann wurde der Druck langsam über 30 min bis zum atmosphärischen Druck entspannt. Das Reaktionsgemisch wurde noch während 2,5 h mit N₂ bei 280 °C überleitet. Der Reaktor wurde anschließend bis Raumtemperatur abgekühlt und 10,1 g Polyamid 12 wurde erhalten.

Scheiben des Polyamidpräparats wurden wie vorstehend beschrieben aus dem erstarrten Schmelzekörper abgedreht. Eine Farbmessung nach DIN 6174 ergab einen b-Wert von 0,2.

Die Scheibe wurde während 24 h im Umlufttrockenschrank bei 100 °C gelagert. Anschließend wurde die Farbe wieder gemessen und das Material wies einen b-Wert von 3,1 und einen ΔE-Wert von 4,0 auf.

### Beispiel B3 (erfindungsgemäß): Herstellung von farblosem Polyamid 12

10 g reines Laurinlactam aus dem Beispiel A3 (20 ppm Verunreinigungen) wurde zusammen mit 10 g Wasser, 0,12 g Dodecandisäure und 0,6 mg H₃PO₂ in einen 25 mL druckfesten Reaktor eingefüllt. Das Gemisch wurde 15 min mit Stickstoff inertisiert und mit einem Metallbad auf 130 °C aufgeheizt. Dann wurde die Temperatur über 30 min bis 280 °C aufgeheizt. Dabei entstand ein Druck von 10 bar. Die Temperatur wurde während 2,5 h bei 280 °C gehalten, wobei ein Druck von 22 bar erreicht wurde. Dann wurde der Druck langsam über 30 min bis zum atmosphärischen Druck entspannt. Das Reaktionsgemisch wurde noch während 2,5 h mit N₂ bei 280 °C überleitet. Der Reaktor wurde anschließend bis Raumtemperatur abgekühlt und 10,1 g Polyamid 12 wurde erhalten.

Scheiben des Polyamidpräparats wurden wie vorstehend beschrieben aus dem erstarrten Schmelzekörper abgedreht. Eine Farbmessung nach DIN 6174 ergab einen b-Wert von 0,2.

Die Scheibe wurde während 24 h im Umlufttrockenschrank bei 100 °C gelagert. Anschließend wurde die Farbe wieder gemessen und das Material wies einen b-Wert von 3,6 und einen ΔE-Wert von 4,3 auf.

### Referenzbeispiel B4: Herstellung von Polyamid 12 aus Laurinlactam mit 530 ppm Verunreinigungen

5 mg Mischung von polyzyklischer Stoffe enthaltend 24 Kohlenstoffatomen mit einer Molmasse zwischen 300 und 380 g/mol (0,2 mg C24H₄₀N, 1,5 mg C₂₄H₄₀O, 1,3 mg C₂₄H₃₉N, 0,3 mg C₂₄H₄₃NO, 0,6 mg C₂₄H₃₈O₂, 0,7 mg C₂₄H₄₂O₂, 0,4 mg C₂₄H₄₂O₂) wurde dem Laurinlactam aus Beispiel A1 zugesetzt. Das resultierende verunreinigte Laurinlactam enthielt 530 ppm Verunreinigungen.

10 g verunreinigtes Laurinlactam (530 ppm Verunreinigungen) wurde zusammen mit 10 g Wasser, 0,12 g Dodecandisäure und 0,6 mg H₃PO₂ in einen 25 mL druckfesten Reaktor eingefüllt. Das Gemisch wurde 15 min mit Stickstoff inertisiert und mit einem Metallbad auf 130 °C aufgeheizt. Dann wurde die Temperatur über 30 min bis 280 °C aufgeheizt. Dabei entstand ein Druck von 10 bar. Die Temperatur wurde während 2,5 h bei 280 °C gehalten, wobei ein Druck von 22 bar erreicht wurde. Dann wurde der Druck langsam über 30 min bis zum atmosphärischen Druck entspannt. Das Reaktionsgemisch wurde noch während 2,5 h mit N₂ bei 280 °C überleitet. Der Reaktor wurde anschließend bis Raumtemperatur abgekühlt und 10,1 g Polyamid 12 wurde erhalten.

Scheiben des Polyamidpräparats wurden wie vorstehend beschrieben aus dem erstarrten Schmelzekörper abgedreht. Eine Farbmessung nach DIN 6174 ergab einen b-Wert von 3,2.

Die Scheibe wurde während 24 h im Umlufttrockenschrank bei 100 °C gelagert. Anschließend wurde die Farbe wieder gemessen und das Material wies einen b-Wert von 12,8 und einen ΔE-Wert von 9,8 auf. Das Material wies bei optischer Betrachtung unter Tageslicht eine deutlich gelbe Farbe auf.

### Referenzbeispiel B5: Herstellung von Polyamid 12 aus Laurinlactam mit 520 ppm Verunreinigungen

5 mg Mischung von polyzyklischer Stoffe enthaltend 24 Kohlenstoffatomen mit einer Molmasse zwischen 300 und 380 g/mol (0,2 mg C₂₄H₄₀N, 1,5 mg C₂₄H₄₀O, 1,3 mg C₂₄H₃₉N, 0,3 mg C₂₄H₄₃NO, 0,6 mg C₂₄H₃₈O₂, 0,7 mg C₂₄H₄₂O₂, 0,4 mg C₂₄H₄₂O₂) wurde dem Laurinlactam aus Beispiel A3 zugesetzt. Das resultierende verunreinigte Laurinlactam enthielt dann 520 ppm Verunreinigungen.

10 g verunreinigtes Laurinlactam (520 ppm Verunreinigungen) wurde zusammen mit 10 g Wasser, 0,12 g Dodecandisäure und 0,6 mg H₃PO₂ in einen 25 mL druckfesten Reaktor eingefüllt. Das Gemisch wurde 15 min mit Stickstoff inertisiert und mit einem Metallbad auf 130 °C aufgeheizt. Dann wurde die Temperatur über 30 min bis 280 °C aufgeheizt. Dabei entstand ein Druck von 10 bar. Die Temperatur wurde während 2,5 h bei 280 °C gehalten, wobei ein Druck von 22 bar erreicht wurde. Dann wurde der Druck langsam über 30 min bis zum atmosphärischen Druck entspannt. Das Reaktionsgemisch wurde noch während 2,5 h mit N₂ bei 280 °C überleitet. Der Reaktor wurde anschließend bis Raumtemperatur abgekühlt und 10,1 g Polyamid 12 wurde erhalten.

Scheiben des Polyamidpräparats wurden wie vorstehend beschrieben aus dem erstarrten Schmelzekörper abgedreht. Eine Farbmessung nach DIN 6174 ergab einen b-Wert von 3,0.

Die Scheibe wurde während 24 h im Umlufttrockenschrank bei 100 °C gelagert. Anschließend wurde die Farbe wieder gemessen und das Material wies einen b-Wert von 13,0 und einen ΔE-Wert von 10,1 auf. Das Material wies bei optischer Betrachtung unter Tageslicht eine deutlich gelbe Farbe auf.

### Ergebnis

Tabelle 1 fasst die b- und ΔE-Werte der fünf Versuche der Beispiele B zusammen.

**Tabelle 1: b- und ΔE-Werte der fünf Versuche.**

| **Beispiel** | **Laurinlactam aus Beispiel...** | **Menge polyzyklischer Stoffe (bezogen auf LL)** | **b-Wert vor Alterung** | **b-Wert nach Alterung** | **ΔE-Wert nach Alterung** |
|---|---|---|---|---|---|
| B1 (erfindungsgemäß) | A1 | 30 ppm | 0,3 | 3,5 | 4,2 |
| B2 (erfindungsgemäß) | A2 | 5 ppm | 0,2 | 3,1 | 4,0 |
| B3 (erfindungsgemäß) | A3 | 20 ppm | 0,2 | 3,6 | 4,3 |
| B4 (Referenz) | A1 | 530 ppm | 3,2 | 12,8 | 9,8 |
| B5 (Referenz) | A3 | 520 ppm | 3,0 | 13,0 | 10,1 |

Polyamide, die aus Laurinlactam mit Verunreinigungen unterhalb von 500 ppm hergestellt wurden, zeigen nach der Alterung nach optischer Betrachtung geringe bzw. keine gelbliche Verfärbung. Ihre b- und ΔE-Werte liegen im Bereich von 3,1 bis 4,3 und damit unterhalb von 5.

Polyamide hingegen, die aus verunreinigtem Laurinlactam mit Verunreinigungen über 500 ppm synthetisiert wurden, zeigen nach Alterung optisch eine deutliche Gelbfärbung. Die b- und ΔE-Werte befinden sich in einem Bereich von 9,8 bis 13,0 und damit deutlich über 5.

## Patentansprüche

1. Verfahren zur Herstellung von Polymeren aus Monomeren enthaltend Laurinlactam, umfassend die Schritte
a. Beckmann-Umlagerung von Cyclododecanoxim zu Laurinlactam in Gegenwart eines Beckmann-Umlagerungskatalysators,
b. Entfernung von Verunreinigungen aus dem Laurinlactam unter Erhalt aufgereinigten Laurinlactams, und
c. Polymerisation von Monomeren umfassend aufgereinigtes Laurinlactam,
wobei die Verunreinigungen polycyclische Stoffe enthaltend 24 Kohlenstoffatome und zumindest ein Heteroatom ausgewählt aus Sauerstoff und Stickstoff umfassen sowie eine Molmasse zwischen 300 und 380 g/mol aufweisen, wobei die Verunreinigungen in Schritt b. auf 500 ppm, bezogen auf Laurinlactam, begrenzt werden.

2. Verfahren nach Anspruch 1, **dadurch gekennzeichnet, dass** die Verunreinigungen mittels Kristallisation oder Destillation entfernt werden.

3. Verfahren nach Anspruch 2, **dadurch gekennzeichnet, dass** die Destillation mit mindestens fünf theoretischen Trennstufen vorgenommen wird.

4. Verfahren nach einem der vorherigen Ansprüche, **dadurch gekennzeichnet, dass** das Cyclododecanonoxim vor der Beckmann-Umlagerung vorbehandelt wird, wobei die Vorbehandlung ausgewählt ist aus Wäsche mit Wasser, Wäsche mit Lauge, Wäsche mit Säure, Hydrierung, Kristallisation oder Kombinationen davon.

5. Verfahren nach einem der vorherigen Ansprüche, **dadurch gekennzeichnet, dass** die Polymerisation kontinuierlich durchgeführt wird.

6. Verfahren nach einem der vorherigen Ansprüche, **dadurch gekennzeichnet, dass** der Beckmann-Umlagerungskatalysator ausgewählt ist aus einer Säure mit pKs ≤ 0, Cyanurchlorid, Phosphortrichlorid, Thionylchlorid oder Mischungen daraus, vorzugsweise Schwefelsäure.

7. Verfahren nach einem der vorherigen Ansprüche, **dadurch gekennzeichnet, dass** die Polymerisation bei einer Temperatur zwischen 250 °C und 350 °C, in Anwesenheit von 2 bis 51 Gew.-% Wasser, bezogen auf das Gesamtgewicht aus Monomeren und Wasser, durchgeführt wird.

8. Verfahren nach einem der vorherigen Ansprüche, **dadurch gekennzeichnet, dass** die Verunreinigungen in Schritt b. auf 100 ppm, bezogen auf Laurinlactam, begrenzt werden.

9. Verfahren nach einem der vorherigen Ansprüche, **dadurch gekennzeichnet, dass** das Polymer mindestens 20 Gew.-% Laurinlactam, bezogen auf das Gesamtgewicht des Polymers, enthält.

10. Polymer enthaltend Laurinlactam, hergestellt nach einem der vorherigen Ansprüche.
